# EUROPÄISCHE PATENTSCHRIFT

(11) **EP 2 108 647 B1**
(45) Veröffentlichungstag und Bekanntmachung des Hinweises auf die Patenterteilung: **21.11.2012**
(21) Anmeldenummer: 09156562.2
(22) Anmeldetag: 30.03.2009
(51) Int. Cl.: C07D 333/28

(54) **Verfahren zur Bromierung von Alkylthiophenen**
Bromination process of 3-alkylthiophenes
Procédé pour la bromuration de 3-alkylthiophènes

(30) Priorität: 11.04.2008 DE 102008018485
(43) Veröffentlichungstag der Anmeldung: 14.10.2009
(73) Patentinhaber: Saltigo GmbH, 40764 Langenfeld (DE)
(72) Erfinder: Steinhaus, Antje, 50733 Köln (DE); Martin, Andreas, 51399 Burscheid (DE); Broda, Witold, 53819 Neunkirchen-Seelscheid (DE)
(74) Vertreter: Pettrich, Klaus-Günter

(56) Entgegenhaltungen:
- CN-A- 101 045 723
- HIGUCHI HIROYUKI ET AL: "SYNTHESIS AND PROPERTIES OF .ALPHA.,.OMEGA.-DISUBSTITUTED OLIGO(3-HEXYLTHIOPHENE)S AND OLIGOTHIENOQUINONOIDS IN HEAD-TO-HEAD ORIENTATION" BULLETIN OF THE CHEMICAL SOCIETY OF JAPAN, CHEMICAL SOCIETY OF JAPAN, TOKYO, JP, Bd. 68, Nr. 8, 1. Januar 1995 (1995-01-01), Seiten 2363-2377, XP008070697 ISSN: 0009-2673

## Beschreibung

Gegenstand der Erfindung ist ein Verfahren zur Bromierung von Alkylthiophenen. Unter Bromierung wird dabei sowohl Di- oder Monobromierung verstanden.

Alkylthiophene, insbesondere 3-Hexylthiophen, werden zur Herstellung organischer halbleitender Polymere, die in elektronischen Bauteilen wie z.B. Halbleitern oder Solarzellen verwendet werden, eingesetzt. Eine Vorstufe zur Herstellung dieser Polyalkylthiophene sind dabei die entsprechenden Mono- oder Dibrom-Derivate.

Die Bromierung von Alkylthiophenen (meist die zweifache Bromierung von 3-Hexyltiophen) wird nach derzeitigem Standard mit Hilfe von elementarem Brom oder unter Einsatz von N-Bromsuccinimid (NBS) durchgeführt.

1988 veröffentlichten Brandsma et al. (Synlett. 1988, 890) die Monobromierung von unsubstituierten Thiophen mit Überschuss HBr und unter stöchiometrischen Mengen an Wasserstoffperoxid. Als Lösungsmittel wurde für diese Reaktion Diethylether, für die Aufarbeitung Pentan eingesetzt. Die Dibromierung von Thiophen wird im selben Artikel beschrieben und mit Hilfe von HBr/Br₂ (ebenfalls in Diethylether) durchgeführt. Burrell et al. (J. Org. Chem. 2003, 8974) beschreiben die Bromierung von 3-Formylthiophen mit HBr/Wasserstoffperoxid.

Die CN101045723 offenbart ein Verfahren zur Bromierung von 3-Methylthiophen mit Bromwasserstoff und Wasserstoffperoxid. Als Lösungsmittel wird Ether eingesetzt, was nach Beendigung der Reaktion eine Extraktion mit Pentan und anschließender Destillation zur Entfernung des Pentans erforderlich macht.

Higuchi Hiroyuki et al. /Bull. Chem. Soc. Jpn., Bd. 68, Nr. 8, 1995, S. 2363-2377) beschreiben ein Verfahren zur Bromierung von 3-Hexylthiophen mit N-Bromsuccinimid in Essigsäure.

Zum Zeitpunkt der Erfindung bestand also immer noch der Bedarf nach einem Verfahren zur Bromierung von Alkylthiophenen, welches den Einsatz von giftigem Brom oder teuren Bromierungsreagenzien vermeidet. Zudem ist auch noch die Raum-Zeit-Ausbeute dieser Reaktion verbesserungswürdig.

Aufgabe der vorliegenden Erfindung war daher, ein Verfahren zur Bromierung von Alkylthiophenen herauszufinden, welches den Einsatz von elementarem Brom (Br₂) oder teuren Bromierungsreagenzien wie N-Bromsuccinimid vermeidet.

Erfindungsgemäß wurde nun gefunden, dass die Bromierung von Alkylthiophenen auch durch den Einsatz von stöchiometrischen Mengen an Bromwasserstoff und Wasserstoffperoxid, ohne dass dabei ein Lösungsmittel zugesetzt werden muss, erfolgen kann.

Gegenstand der Erfindung ist daher ein Verfahren zur Mono- oder Dibromierung von Alkylthiophen, bei dem ein Alkylthiophen der allgemeinen Formel (I) 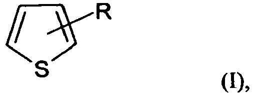 wobei
- R: für einen verzweigten oder unverzweigten Alkylrest mit 1 bis 12 C-Atomen steht,
mit Mengen an HBr und H₂O₂, in Kontakt bringt, die man zur Herstellung des Mono- bzw. Dibromproduktes benötigt, dadurch gekennzeichnet, dass das HBr eine wässrige HBr-Lösung mit einem Gehalt von 10 bis 62 Gew.-% Bromwasserstoffsäure ist, das H₂O₂ eine wässrige H₂O₂-Lösung mit einem Gehalt von 25 bis 35 Gew.-% Wasserstoffperoxid ist und das Verfahren ohne Zusatz eines Lösungsmittels durchgeführt wird.

Die Bromierung des Alkylthiophens wird dabei als Mono- oder Dibromierung durchgeführt. Bevorzugt wird die Bromierung als Dibromierung durchgeführt, d.h. dass 2 Kohlenstoffatome des Thiophen-Gerüsts bromiert werden. Gesteuert wird der Grad der Bromierung dabei durch den entsprechenden stöchiometrischen Einsatz an HBr und H₂O₂. Unter einer stöchiometrischen Menge wird dabei die Menge an HBr und H₂O₂ verstanden, die man zur Herstellung des Mono- bzw. Dibromproduktes benötigt.

Üblicherweise setzt man das HBr dabei als wässrige HBr-Lösung mit einem Gehalt von 10 bis 62 Gew.-% Bromwasserstoffsäure, bevorzugt 30 bis 60 Gew.%, besonders bevorzugt 48 Gew.-% Bromwasserstoffsäure ein. Als H₂O₂ wird eine wässrige H₂O₂-Lösung mit einem Gehalt von 25 bis 35 Gew.-% Wasserstoffperoxid, bevorzugt 30-35 Gew.-% Wasserstoffperoxid eingesetzt.

Die Reaktionsdauer, in der Alkylthiophen mit HBr und H₂O₂ in Kontakt gebracht wird, liegt üblicherweise zwischen 1 und 36 Stunden, vorzugsweise zwischen 5 und 10 Stunden.

Die Reaktionstemperatur liegt üblicherweise im Bereich zwischen -10 und +80 °C, bevorzugt zwischen -5 und +50 °C, besonders bevorzugt bei 45 °C.

Das erfindungsgemäße Verfahren wird ohne Zusatz eines weiteren Lösungsmittels durchgeführt.

Mit dem erfindungsgemäßen Verfahren lassen sich alle Stellungsisomere von Alkylthiophen bromieren. Bevorzugt wird aber 3-Alkylthiophen mit 1 bis 12 C-Atomen in der Alkylkette im erfindungsgemäßen Verfahren eingesetzt. Besonders bevorzugt wird das 3-Hexylthiophen im erfindungsgemäßen Verfahren zur Bromierung eingesetzt, wobei bevorzugt das Mono- bzw. Dibromprodukt hergestellt wird.

Die nachfolgenden Beispiele sollen die Erfindung weiter erläutern, ohne sie jedoch in ihrem Umfang zu begrenzen.

### Beispiele

### Beispiel 1 (vergleichend)

75 g 3-Hexylthiophen wurden in 375 mL THF vorgelegt. Bei RT werden 167 g NBS portionsweise binnen 4 h zugegeben. Nach 17 stündigem Nachrühren wurden 16 g NBS und 25 mL THF nachdosiert und weitere 17 h nachgerührt. Es wurde filtriert und das Lösungsmittel abdestilliert. Das Rohprodukt (2,5-Dibrom-3-hexylthiophen) hatte eine Reinheit von 91,7%, die Ausbeute des Rohprodukts betrug 83%.

### Beispiel 2 (erfindungsgemäß)

338 g 3-Hexylthiophen wurden mit 1070 g 48%iger Bromwasserstoffsäure vorgelegt. Es wurde auf -5 °C gekühlt und über einen Zeitraum von 7 h mit 400 g 34%igem Wasserstoffperoxid versetzt. Innerhalb von 16 h wurde auf 20 °C erwärmt. Die Phasen wurden getrennt. Das Rohprodukt (2,5-Dibrom-3-hexylthiophen) hatte eine Reinheit von 96,9%, die Ausbeute des Rohprodukts betrug 97%.

### Beispiel 3 (erfindungsgemäß)

343 g Hexylthiophen wurden mit 674 g 48%iger Bromwasserstoffsäure vorgelegt. Es wird auf 37 °C erwärmt und über einen Zeitraum von 7 h mit 454 g 30%igem Wasserstoffperoxid versetzt. Es wurde über Nacht bei 45 °C gerührt. Dann wurden 21 g 40%ige Natriumbisulfitlösung zugegeben und die Phasen getrennt. Das Rohprodukt hatte eine Reinheit von 90,7%, die Ausbeute des Rohprodukts (2,5-Dibrom-3-hexylthiophen) betrug 88%.

## Patentansprüche

1. Verfahren zur Mono- oder Dibromierung von Alkylthiophen, bei dem ein Alkylthiophen der allgemeinen Formel (I) 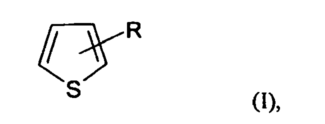 wobei
R für einen verzweigten oder unverzweigten Alkylrest mit 1 bis 12 C-Atomen steht,
mit Mengen an HBr und H₂O₂, in Kontakt bringt, die man zur Herstellung des Mono- bzw. Dibromproduktes benötigt, **dadurch gekennzeichnet, dass** das HBr eine wässrige HBr-Lösung mit einem Gehalt von 10 bis 62 Gew.-% Bromwasserstoffsäure ist, das H₂O₂ eine wässrige H₂O₂-Lösung mit einem Gehalt von 25 bis 35 Gew.-% Wasserstoffperoxid ist und das Verfahren ohne Zusatz eines Lösungsmittels durchgeführt wird.

2. Verfahren nach Anspruch 1, **dadurch gekennzeichnet, dass** das Alkylthiophen der allgemeinen Formel (I) mit dem HBr und dem H₂O₂ bei einer Temperatur im Bereich von - 10 bis +80°C in Kontakt gebracht wird.

3. Verfahren nach einem der Ansprüche 1 bis 2, **dadurch gekennzeichnet, dass** das Alkylthiophen der allgemeinen Formel (I) ein 3-Alkylthiophen ist.

4. Verfahren nach einem der Ansprüche 1 bis 3, **dadurch gekennzeichnet, dass** das Alkylthiophen 3-Hexylthiophen ist und das Produkt 2-Brom-3-hexylthiophen oder 2,5-Dibrom-3-hexylthiophen ist.

## Claims

1. Process for mono- or dibrominating alkylthiophene, in which an alkylthiophene of the general formula (I) 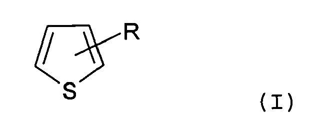 where
R is a branched or unbranched alkyl radical having 1 to 12 carbon atoms
is contacted with amounts of HBr and H₂O₂ which are required to prepare the mono- or dibromo product, **characterized in that** the HBr is an aqueous HBr solution having a content of 10 to 62% by weight of hydrobromic acid, the H₂O₂ is an aqueous H₂O₂ solution having a content of 25 to 35% by weight of hydrogen peroxide and the process is carried out without addition of a solvent.

2. Process according to Claim 1, **characterized in that** the alkylthiophene of the general formula (I) is contacted with the HBr and the H₂O₂ at a temperature in the range of -10 to +80°C.

3. Process according to either of Claims 1 and 2, **characterized in that** the alkylthiophene of the general formula (I) is a 3-alkylthiophene.

4. Process according to any one of Claims 1 to 3, **characterized in that** the alkylthiophene is 3-hexylthiophene and the product is 2-bromo-3-hexylthiophene or 2,5-dibromo-3-hexylthiophene.

## Revendications

1. Procédé pour la mono- ou dibromation d'alkylthiophène, dans lequel on met en contact un alkylthiophène de formule générale (I) 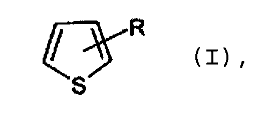 dans laquelle
R représente un radical alkyle ramifié ou non ramifié ayant de 1 à 12 atomes de carbone,
avec les quantités de HBr et H₂O₂ dont on a besoin pour la préparation du produit mono- ou, respectivement, dibromé, **caractérisé en ce que** HBr est une solution aqueuse de HBr ayant une teneur en acide bromhydrique de 10 à 62 % en poids, le H₂O₂ est une solution aqueuse de H₂O₂ ayant une teneur en peroxyde d'hydrogène de 25 à 35 % en poids et le procédé est effectué sans addition d'un solvant.

2. Procédé selon la revendication 1, **caractérisé en ce qu'**on met l'alkylthiophène de formule générale (I) en contact avec le HBr et le H₂O₂ à une température dans la plage de -10 à +80 °C.

3. Procédé selon la revendication 1 ou 2, **caractérisé en ce que** l'alkylthiophène de formule générale (I) est un 3-alkylthiophène.

4. Procédé selon l'une quelconque des revendications 1 à 3, **caractérisé en ce que** l'alkylthiophène est le 3-hexylthiophène et le produit est le 2-bromo-3-hexylthiophène ou le 2,5-dibromo-3-hexylthiophène.
